# EUROPEAN PATENT APPLICATION

(11) **EP 1 752 091 A1**
(43) Date of publication of application: **14.02.2007**
(21) Application number: 06016584.2
(22) Date of filing: 08.08.2006
(51) Int. Cl.: A61B 5/022

(54) **Electronic blood pressure monitor**

(30) Priority: 12.08.2005 JP 2005234871
(71) Applicant: Omron Healthcare Co., Ltd., Kyoto 615-0084 (JP)
(72) Inventor: Sawanoi, Yukiya Omron Healthcare Co., Ltd., Kyoto-shi Kyoto 615-0084 (JP); Kishimoto, Hiroshi Omron Healthcare Co., Ltd., Kyoto-shi Kyoto 615-0084 (JP); Matsumura, Naomi Omron Healthcare Co., Ltd., Kyoto-shi Kyoto 615-0084 (JP)
(74) Representative: Kilian, Helmut

(57) **Abstract**

An electronic blood pressure monitor (1) has a switch (21) detecting whether a front cover is open or closed. When the front cover is opened by a subject, the switch (21) detects that the cover is opened, and outputs a detection signal to a control portion (16). In control portion (16) a CPU (161) operates in response to the detection signal being received to cause a power supply switch LED (51) to flash on and off/turn on. This indicates that of a variety of switches of a console (4), the type of manipulation switch that should first be operated is a power supply switch (41).

## Description

### BACKGROUND OF THE INVENTION

### Field of the Invention

The present invention relates generally to electronic blood pressure monitors and particularly to electronic blood pressure monitors having a function guiding which manipulation switch should be operated.

### Description of the Background Art

Currently, domestic pressure measuring instruments are rapidly increasingly used. Blood pressure is one index employed to analyze circulatory diseases, and analyzing a risk based on blood pressure is effective for example in preventing cerebral apoplexy, cardiac failure, myocardial infarction and other cardiovascular diseases. Such risk analysis needs to be based on a result of measuring blood pressure over a long period of time of at least one week under a fixed condition. Accordingly, electronic blood pressure monitors have been proposed that have a function to record results obtained from measuring blood pressure a plurality of times. Some proposed electronic blood pressure monitors have a function to record results of measuring a plurality of people for blood pressure, a function to record results of measuring blood pressure in the morning, in the evening or for each condition for measurement, and the like.

A conventional blood pressure monitor, for example a blood pressure monitor capable of recording obtained measurements of a plurality of people, allows a user to provide an input via a switch before or after measurement to designate a person to be measured. Similarly, a blood pressure monitor that records an obtained measurement for each condition for measurement also allows a user to input a condition for measurement via a switch before or after measurement to designate the condition for measurement.

Designating a subject, a condition for measurement and the like, as described above depends on the subject performing a series of operations to do so. If the subject forgets to perform an operation designating the subject, the condition for measurement and the like, and thus measures blood pressure and obtains a measurement, the obtained measurement cannot be recorded for the subject, the condition for measurement and the like, and such obtained measurement recorded cannot provide a correct disease risk analysis. To prevent a subject from forgetting to perform such operation, there is also provided a blood pressure monitor having a function to display an operation procedure on a liquid crystal display. However, it has a display and a manipulation switch remote from each other. This renders it difficult for a user to immediately recognize a manipulation switch indicated in the operation procedure displayed on the display. As such, the function fails to serve to effectively prevent erroneous operation.

Japanese Utility Model Laying-Open No. 62-130609 discloses an electronic blood pressure monitor including a main body and a protection cover covering the main body. As a user opens the protection cover, an initial step of blood pressure measurement is indicated, and in response, supplying each component of the main body with power starts. Automatically supplying power in response to the protection cover being opened can eliminate the necessity of the user operating a switch to start to supply power. However, the disclosed electronic blood pressure monitor does not inform the user of which switch should be operated after starting to supply power. As a result, the user does not know which switch should next be operated. Furthermore, a user unfamiliar with the disclosed electronic blood pressure monitor may try to operate a switch to start to supply power despite that the blood pressure monitor has already been supplied with power. The disclosed blood pressure monitor is thus poor in operability.

### SUMMARY OF THE INVENTION

The present invention contemplates an electronic blood pressure monitor capable of guiding in a correct operation procedure.

To achieve the above object the present invention in an aspect provides an electronic blood pressure monitor including: a cuff attached at a site for measurement of blood pressure; a pressure adjustment portion adjusting a pressure applied to the cuff; a pressure detection portion detecting a pressure internal to the cuff; a blood pressure calculation portion calculating a blood pressure based on a detection signal output from the pressure detection portion; a storage portion storing to a memory the blood pressure thus calculated; a display at least displaying the blood pressure thus calculated; and a plurality of manipulation portions each externally operated to indicate an operation of the electronic blood pressure monitor. To indicate the operation, an order of operating the manipulation portion to be operated is indicated in a predetermined manner.

As the subject is informed of an order of operating the manipulation portions, the subject can operate the manipulation portions in the order. This can prevent the subject from performing erroneous operation.

Preferably the electronic blood pressure monitor further includes a cover covering at least a portion of the electronic blood pressure monitor and opened and closed as desired. When the cover is opened, one of the plurality of manipulation portions that should first be operated is indicated in the predetermined manner. Thus when the subject opens the cover to use the electronic blood pressure monitor, the electronic blood pressure monitor responsively indicates in a predetermined manner a manipulation portion to be first operated. This can prevent the subject from performing erroneous operation.

Preferably the cuff is detachably attachable to the electronic blood pressure monitor, and the electronic blood pressure monitor further includes a cuff detection portion detecting whether the electronic blood pressure monitor has the cuff attached thereto. When the cuff detection portion detects that the electronic blood pressure monitor has the cuff attached thereto, one of the plurality of manipulation portions that should first be operated is indicated in the predetermined manner. Thus when a subject attaches the cuff to the electronic blood pressure monitor to use it the electronic blood pressure monitor responsively indicates in a predetermined manner a manipulation portion to be first operated. This can prevent the subject from performing erroneous operation.

Preferably the electronic blood pressure monitor further includes a detection portion detecting that the cuff has been attached to the site for measurement. When the detection portion detecting that the cuff has been attached to the site for measurement detects that the cuff has been attached to the site for measurement, one of the plurality of manipulation portions that should first be operated is indicated in the predetermined manner. Thus when the cuff is attached to the site for measurement to use the electronic blood pressure monitor the electronic blood pressure monitor responsively indicates in a predetermined manner a manipulation portion to be first operated. This can prevent the subject from performing erroneous operation.

Preferably if the manipulation portion is operated in an order different from the order indicated, the manipulation portion that should be operated in the order indicated is indicated in the predetermined manner. Thus if a subject operates a manipulation portion in an erroneous operation order the subject can be informed of a manipulation portion to be operated in a correct operation order. The subject can thus be informed that the subject has performed an erroneous operation. Furthermore, the subject can be urged to operate a manipulation portion in the correct operation order.

Preferably, the order indicated is indicated by illuminating the manipulation portion. Thus a manipulation portion to be operated is illuminated and the subject can immediately confirm that which manipulation portion should be operated.

Preferably the electronic blood pressure monitor according to claim 8, further includes an illuminator associated with each of the plurality of manipulation portions and provided to be integrated therewith for illuminating the manipulation portion. The illuminator integrated with a manipulation portion allows the electronic blood pressure monitor to be compact and can also illuminate the manipulation portion effectively.

Preferably the illuminator is arranged in a vicinity of the manipulation portion. Thus a manipulation portion to be operated can be illuminated from a vicinity thereof.

Preferably an illuminator associated with a manipulation portion to be operated in the indicated order is turned on or flashed on and off to indicate the order.

Preferably the electronic blood pressure monitor further includes an information displaying portion associated with a manipulation portion. The indicated order is displayed via the information displaying portion. The subject can thus be informed, by information displayed, of which manipulation portion should be operated.

Preferably the information displaying portion and a manipulation portion associated therewith are integrally configured. The electronic blood pressure monitor can thus be compact.

Preferably the information displaying portion is arranged in a vicinity of the manipulation portion associated therewith. This allows a subject to confirm, by predetermined information displayed in the vicinity of the manipulation portion, that the manipulation portion is that which should be operated in the indicated order.

Preferably the electronic blood pressure monitor further includes a joint jointing a cover and a main body of the electronic blood pressure monitor together and the cover is jointed via the joint to the main body pivotably to be opened and closed as desired. Whether the cover is opened or closed is detected from whether it is pivoted. How the cover is pivoted at the joint to be opened and closed can be utilized to obtain a timing to indicate a manipulation portion to be first operated.

Preferably whether the main body of the electronic blood pressure monitor and the cover contact each other or not is detected to detect whether the cover is open or closed. The fact that the cover and the main body contact or does not contact each other as the cover is opened or closed, can be utilized to obtain a timing of indicating a switch to be first operated.

In accordance with the present invention an order of operating manipulation portions can be indicated and a subject can follow the order to operate a manipulation portion. This can prevent the subject from performing erroneous operation. This can in turn prevent an obtained measurement, or a calculated blood pressure, from being associated with a wrong subject and thus stored to memory, and associated with a wrong condition for measurement and thus stored to memory.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Figs. 1-3 show in appearance an electronic blood pressure monitor in embodiments, respectively.
Fig. 4 shows a configuration in function of the electronic blood pressure monitor in a first embodiment.
Fig. 5 shows an example of contents of a memory in each embodiment.
Fig. 6 is a flowchart of a process performed by the electronic blood pressure monitor in the first embodiment.
Fig. 7 shows a configuration in function of the electronic blood pressure monitor in a second embodiment.
Fig. 8 is a flowchart of a process performed by the electronic blood pressure monitor in the second embodiment.
Figs. 9A and 9B show one example in configuration of a cuff connection detection switch.
Figs. 10A and 10B show another example in configuration of the cuff connection detection switch.
Fig. 11 shows a configuration in function of the electronic blood pressure monitor in a third embodiment.
Fig. 12 is a flowchart of a process performed by the electronic blood pressure monitor in the third embodiment.
Fig. 13 shows one example in configuration of a switch provided to detect whether a cuff is wounded.
Figs. 14-18 each show a specific example of indicating an operation procedure.
Figs. 19 shows in appearance the electronic blood pressure monitor in still another embodiment.
Fig. 20 shows a configuration in function of the electronic blood pressure monitor in still another embodiment.
Figs. 21 shows in appearance the electronic blood pressure monitor in still another embodiment.

### DESCRIPTION OF THE PREFERRED EMBODIMENTS

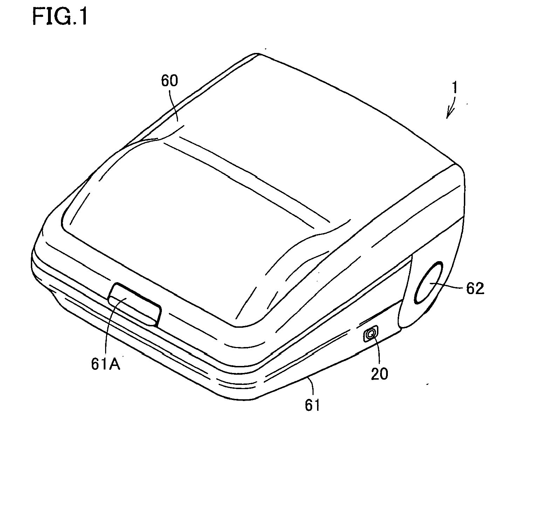

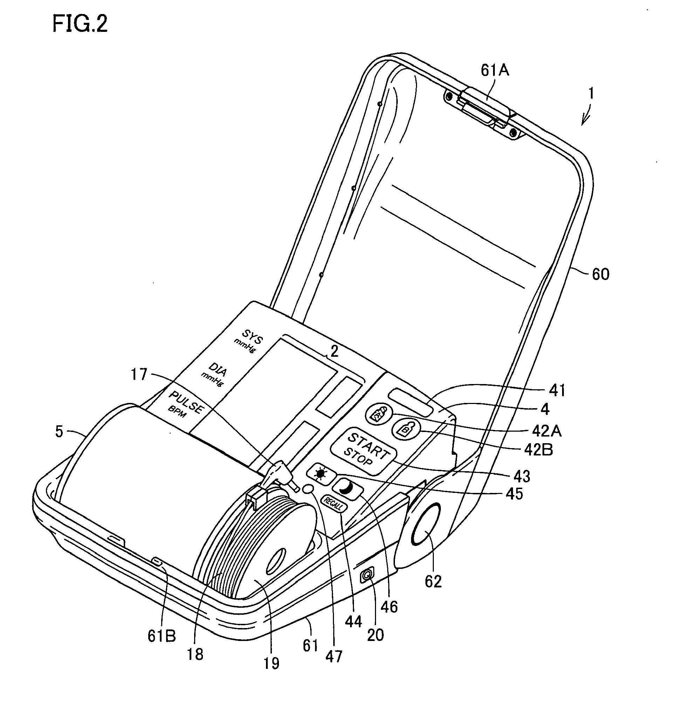

Figs. 1-3 show a configuration of an electronic blood pressure monitor 1 in each embodiment that can be shared by a plurality subjects. Electronic blood pressure monitor 1 includes a main body 61, a front cover 60 provided to cover a surface of main body 61 for protection, and a joint 62 jointing front cover 60 and main body 61 together. Front cover 60, pivoting around joint 62, is opened and closed, as desired, relative to main body 61, as shown in Figs. 1 and 2.

Front cover 60 may be formed of transparent material allowing a surface of main body 61 to be externally observable with front cover 60 closed as shown in Fig. 1, or may be formed of opaque material. Front cover 60 formed of transparent material is convenient for conforming what (i.e., blood pressure being measured) is displayed on a display 2 when blood pressure is measured with front cover 60 closed.

Furthermore, main body 61 includes a connector portion 20 located in a side surface of the main body for feeding electronic blood pressure monitor 1 with power, and an air connector 31 (not shown) located at an opposite side surface for supplying/exhausting air in measuring blood pressure.

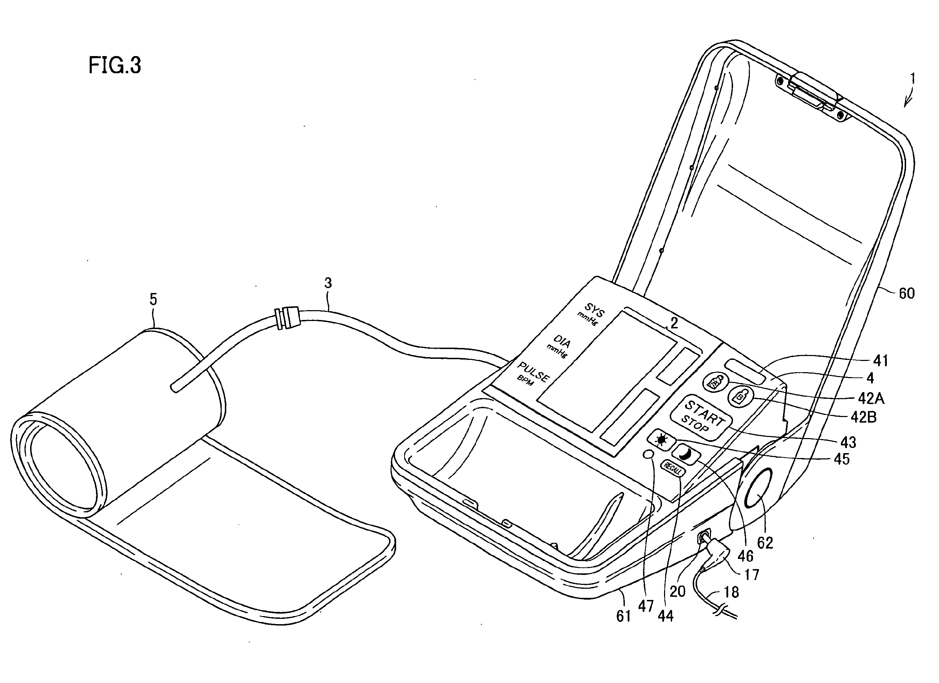

Fig. 3 shows a manner for use of electronic blood pressure monitor 1 in measuring blood pressure. To measure blood pressure, a subject opens front cover 60 (see Fig. 2) and extracts from a front portion of main body 61 a cord reel 19 having a power supply cord 18 would therearound, and a cuff 5. Then, as shown in Fig. 3, power supply cord 18 is unwound from cord reel 19, and a plug 17 of one end of power supply cord 18 is plugged into connector portion 20 and a plug (not shown) of the other end of power supply cord 18 of cord reel 19 is inserted into a plug socket of an alternate current (AC) power supply (a commercial power supply) (not shown). Electronic blood pressure monitor 1 is thus supplied with power from the AC power supply through power supply cord 18.

Cuff 5 is connected to an air tube 3 having one end connected to an air bag (not shown) internal to cuff 5 and the other end inserted into air connector 31 (not shown) of main body 61. Thus through air tube 3 main body 61 can supply cuff 5 (or the air bag) with air and exhaust air from cuff 5.

Electronic blood pressure monitor 1 has cuff 5 and power supply cord 18 detachably attachable to main body 61 and hence separate from main body 61. Alternatively, cuff 5 alone may be provided as a separate component and main body 61 and power supply cord 18 may integrally be provided. Furthermore, front cover 60 provided to cover the entire surface of main body 61 may alternatively be provided to cover only a portion of the front surface portion excluding that accommodating cord reel 19 having power supply cord 18 wound therearound and cuff 5.

Main body 61 has a casing with a surface provided with a console 4 operable by a user and display 2 displaying an obtained measurement or the like to allow the user to confirm what is displayed.

Console 4 includes a power supply switch 41, a user selection switch group 42 configured of user selection switches 42A and 42B, a measuring switch 43 operated to start or stop measurement, a recalling switch 44 operated to recall data of a obtained blood pressure measurement stored in main body 61 at a memory 13 as described later, a morning switch 45 and an evening switch 46 operated to indicate whether data to be recalled is a measurement obtained in the morning or that obtained in the evening, and a clock setting switch 47 operated to set a time counted by a timer 14 as described later. The variety of switches of console 4 is formed of light guiding material, such as acrylic resin.

Herein, recalling data indicates reading data from memory 13 and displaying the read data on a display 2.

Console 4 has each switch with a back side provided with a light emitting diode (LED) or a similar illuminator (not shown) associated with the switch. The illuminator emits light which is radiated externally through the light guiding material of the switch associated therewith. Thus employing the illuminator allows the switch to emit light.

In each embodiment a subject (or user) is guided through a correct blood pressure measurement operation procedure as the subject is informed of (or guided through) an order of operating a switch of console 4 that should be operated. The subject is informed of the order by the illuminator (a back light) provided at a back side of each switch and controlled to flash on and off or turn on, as has been described hereinbefore.

### First Embodiment

In a first embodiment, upon opening front cover 60, the electronic blood pressure monitor start informing an order of operating a switch to be operated.

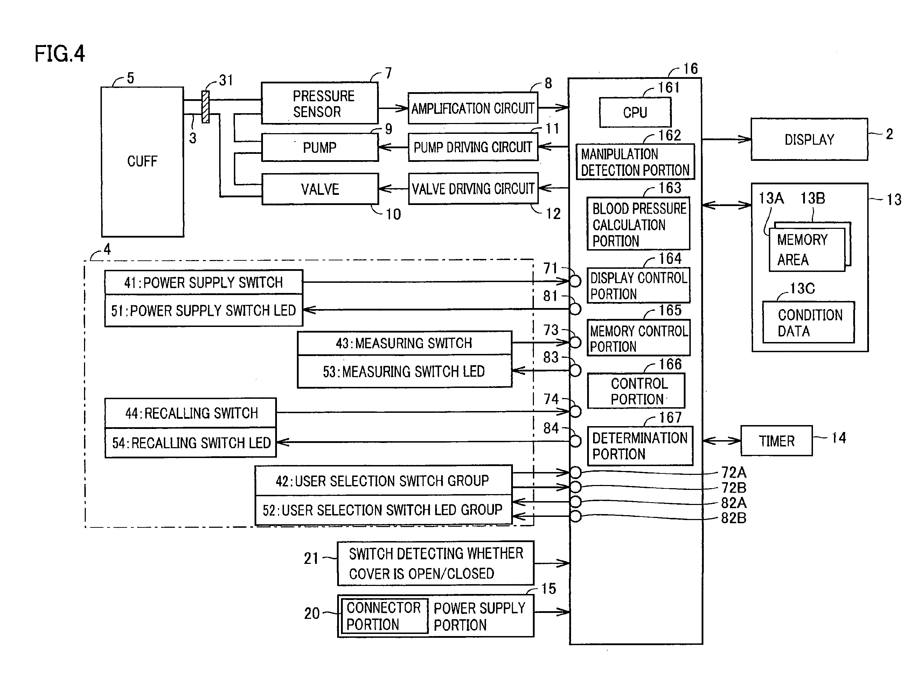

With reference to Fig. 4 the present embodiment provides electronic blood pressure monitor 1 including cuff 5 attached on a subject at a site (an upper arm) for measurement of blood pressure for applying pressure by air pressure, and air tube 3 connecting main body 61 and cuff 5 together. In Fig. 4, a portion rightward of air connector 31 indicates each component corresponding to main body 61. Main body 61 includes display 2 provided to allow a subject to confirm what is displayed, and console 4 provided to be operable externally by the subject. Herein the site for measurement is an upper arm, however it is not limited thereto, and it may be a wrist or a finger.

Main body 61 includes a pressure sensor 7 outputting in the form of a pulse wave signal a variation in pulse pressure detected at a site for measurement via the air bag (not shown) incorporated in cuff 5, an amplification circuit 8 amplifying a voltage signal indicative of a pressure output from pressure sensor 7, a pump 9 and a valve 10 for adjusting in level a pressure (an air pressure) applied by the air bag to the site for measurement, a pump driving circuit 11 for driving pump 9, a valve driving circuit 12 adjusting how valve 10 is opened/closed, memory 13, timer 14 operating to count time and outputting data of time counted, a power supply portion 15 having connector portion 20 to supply each component of main body 61 with power provided through connector portion 20, a control portion 16, and a switch 21 detecting that front cover 60 is opened/closed. Control portion 16 has a central processing unit (CPU) 161 controlling each component of main body 61, and input ports 71, 72A, 72B, 73 and 74 and output ports 81, 82A, 82B, 83 and 84. Furthermore, control portion 16 includes a manipulation detection portion 162, a blood pressure calculation portion 163, a display control portion 164, a memory control portion 165, a control portion 166 controlling the illuminator to flash on and off, turn on, and the like, and a determination portion 167 determining from a detection signal provided from switch 21 whether front cover 60 is opened or closed. Note that determination portion 167 has a function serving as a detection portion detecting a timing of starting an operation.

Manipulation detection portion 162 detects (or determines) which type of switch of console 4 has been operated or whether any switch has been operated. Blood pressure calculation portion 163 calculates blood pressure and pulse. Display control portion 164 controls display 2 to operate for displaying. Memory control portion 165 has a function to access memory 13. The functions of manipulation detection portion 162, blood pressure calculation portion 163, display control portion 164, memory control portion 165, control portion 166 and determination portion 167 are implemented by CPU 161 reading and executing a predetermined program previously stored in memory 13. While each portion's function is herein implemented by a program, the entirety or a portion thereof may be implemented by a circuit or other similar hardware.

Clock setting switch 47 can be operated to externally set time count data of timer 14.

Power supply 15 has a rechargeable battery (not shown). When connector portion 20 does not have power supply cable 18 connected thereto, power supply portion 15 supplies power from the battery to control portion 16 including CPU 161. When connector portion 20 has power supply cable 18 connected thereto, through power supply cable 18 an external ac power supply supplies power, which is supplied by power supply portion 15 to each component of main body 61 and also used to charge the battery. Note that power supply portion 15 having the rechargeable battery may alternatively be implemented by an alkaline battery. In that case, while connector portion 20 does not have power supply cable 18 connected thereto, the alkaline battery supplies power to control portion 16 including CPU 161. When connector portion 20 has power supply cable 18 connected thereto, through power supply cable 18 an external ac power supply supplies power, which is supplied by power supply portion 15 to each component of main body 61, and the alkaline battery is electrically disconnected from each component of main body 61.

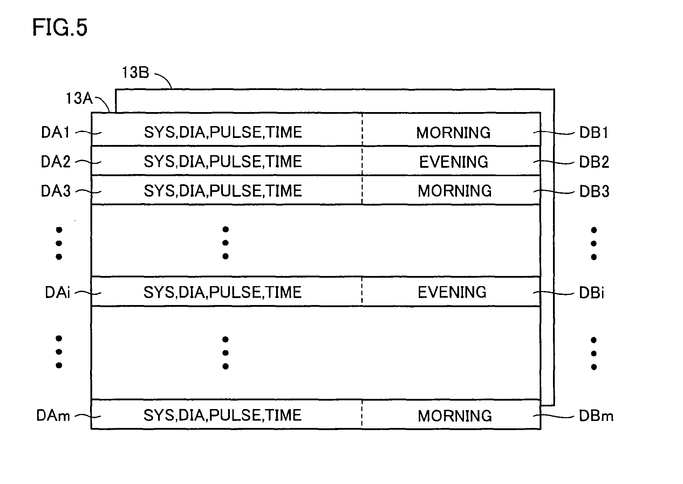

Memory 13 has an individual memory area for an obtained measurement for each user to allow electronic blood pressure monitor 1 to be shared by a plurality of people. Herein, electronic blood pressure monitor 1 is shared by two persons for the sake of illustration. Accordingly, memory 13 has areas 13A and 13B each for storing an obtained measurement for a user (or a subject). As shown in Fig. 5, memory areas 13A and 13B each store data DAi indicating an obtained blood pressure measurement and data DBi indicating a condition for measurement such that data DAi and DBi are associated with each other, wherein i = 1, 2, 3, ..., i, ..., m. Data DAi includes data of systolic blood pressure (mmHg) indicated in the figure as "SYS", diastolic blood pressure (mmHg) indicated in the figure as "DIA", a pulse rate per minute indicated in the figure as "PULSE", and measurement time indicated in the figure as "TIME". For example, data DBi indicates whether a subject measured blood pressure in the morning or in the evening. Alternatively, the data indicates whether a subject measured blood pressure after getting up or before going to bed. Alternatively, the data indicates whether a subject measured blood pressure before or after the subject had drug administration. Alternatively, the data indicates whether a subject measured blood pressure before or after exercise. Alternatively, the data indicates an weekend or a week day. Herein, by way of example, morning/evening is assumed as a condition for measurement.

The condition for measurement "morning/evening" is temporarily stored in memory 13 as condition data 13C. If CPU 161 determines that measurement time data received from timer 14 corresponds to a predetermined period of time corresponding to the morning, condition data 13C is set to indicate "morning", and if CPU 161 determines that measurement time data received from timer 14 corresponds to a predetermined period of time corresponding to the evening, then condition data 13C is set to indicate "evening". Note that the data of the predetermined period of time indicating the morning and that of the predetermined period of time indicating the evening are assumed to be previously stored in memory 13. Thus CPU 161 compares the measurement time data received from timer 14 with the data of the predetermined period of time read from memory 13 to determine a condition for measurement.

With reference to Figs. 3 and 4, console 4 includes power supply switch 41, user selection switches 42A and 42B operated to distinguish subjects, measuring switch 43 operated to indicate whether to start or stop pressurization (or measurement), clock setting switch 47, recalling switch 44 operated to recall data of an obtained measurement stored, and morning switch 45 and evening switch 46.

Morning switch 45 and evening switch 46 are operated to designate data DAi of an obtained measurement that should be to be recalled. More specifically, the switches are operated to designate whether the data to be recalled is data DAi of an obtained measurement with the corresponding data DBi of a condition for measurement indicating "morning" or data DAi of an obtained measurement with the corresponding data DBi of a condition for measurement indicating "evening".

When user selection switch 42A or 42B is operated, in response to which switch having been operated, either memory area 13A or 13B is designated as an area that should store data of an obtained measurement or an area searched to recall such data. When user selection switch 42A is operated memory area 13A is designated and when user selection switch 42B is operated memory area 13B is designated.

Power supply switch 41 is operated to start or stop supplying power from power supply portion 15 to each component (excluding control portion 16) for operation, as will be described later. As such, if, in operation, power supply switch 41 is operated, the operation is stopped (or terminated).

In Fig. 4 at console 4 only power supply switch 41, user selection switch group 42 formed of user selection switches 42A and 42B (not shown), measuring switch 43 and record recalling switch 44 are shown for the sake of illustration. At back sides of power supply switch 41, user selection switch group 42, measuring switch 43 and recalling switch 44, respectively, a power supply switch LED 51, a user selection switch LED group 52, a measuring switch LED 53 and a recalling switch LED 54 are provided, respectively, such that the LEDs are integrated with their respective switches. User selection switch LED group 52 has user selection switch LEDs (A) and (B) (not shown) at back sides of user selection switches 42A and 42B, respectively, such that the LEDs are integrated with the switches, respectively.

Control portion 16 has input ports 71-74 and output ports 81-84 paired therewith, respectively, as has been described hereinbefore.

Input port 71 is connected to power supply switch 41 to receive a signal output when power supply switch 41 is operated. Input ports 72A and 72B are connected to user selection switches 42A and 42B, respectively, to receive signals output when their respective switches are operated. Input port 73 is connected to measuring switch 43 to receive a signal output when measuring switch 43 is operated. Output port 81 is connected to power supply switch LED 51 and outputs a signal instructing power supply switch LED 51 to flash on and off or turn on. Output ports 82A and 82B are connected to user selection switch LEDs (A) and (B) of user selection switch LED group 52 and each output a signal instructing a switch LED connected thereto to flash on and off or turn on. Output port 83 is connected to measuring switch LED 53 and outputs a signal instructing measuring switch LED 53 to turn on or flash on and off. Output port 84 is connected to recalling switch LED 54 and outputs a signal instructing recalling switch LED 54 to flash on and off or turn on. Control portion 166 provides each output port with a signal of an instruction that the output port should output.

Input ports 71-74 each receive a signal in the form of a pulse signal having a width of a predetermined period of time. More specifically, when a corresponding switch is operated, the pulse signal transitions from "0" to "1" and thereafter for only a short, predetermined period of time holds the level of "1" and transitions to "0" after that predetermined period of time elapses. If manipulation detection portion 162 detects that any input port receives the pulse signal, manipulation detection portion 162 determines (or detects) that the switch connected to that port has been operated.

Switch 21 detects whether front cover 60 is open or closed and outputs a signal indicative of a resultant detection to detection portion 167.

Switch 21 turns on/off as a cam (not shown) incorporated in joint 62 moves. The cam operates as front cover 60 is opened and closed (or pivoted) around joint 62. Thus a signal can be output that indicates whether switch 21 is turned on (i.e., front cover 60 is open) or switch 21 is turned off (i.e., front cover 60 is closed).

Determination portion 167 determines from a signal received from switch 21 whether front cover 60 is opened or closed.

While herein switch 21 is described as a type of cam switch, it is not limited thereto. For example, a function provided to detect whether main body 61 and front cover 60 contact each other may be provided at such an area that when front cover 60 assumes a closed position main body 61 and front cover 60 contact each other and when front cover 60 assumes an opened position main body 61 and front cover 60 do not contact each other. For example, it may be implemented by a switch 61B turned on when front cover 60 is opened, as shown in Fig. 2, and a magnet 61A. When front cover 60 is closed as shown in Fig. 1, switch 61B is attracted by and thus closely contacts magnet 61A and thus switched off.

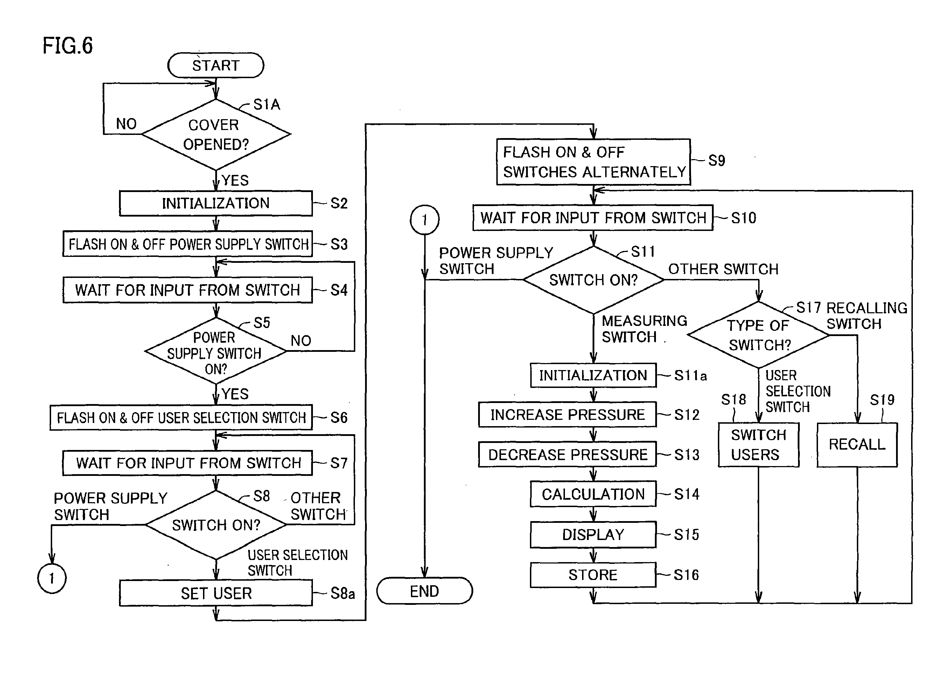

Fig. 6 is a flowchart showing a procedure for informing (or guiding) a user of (or through) an order of operating a switch to correctly operate electronic blood pressure monitor 1 of the present embodiment (to measure blood pressure and recall data of a measurement). The Fig. 6 flowchart is stored previously as a program in memory 13 at a predetermined area and read and executed by CPU 161.

Note that it is assumed that to measuring blood pressure a subject connects air tube 3 to air connector 31 and has cuff 5 wound around a site for measurement. Furthermore it is assumed that the subject inserts plug 17 into connector portion 20 and connects power supply cable 18 to ac power supply to allow main body 61 to have each component with power supplied thereto. In recalling blood pressure measurement data, while the subject may connect air tube 3 to air connector 31, the subject may not wind cuff 5 around the site for measurement.

In such condition, determination portion 167 determines from a level of a signal received from switch 21 whether front cover 60 is open or closed (step (S) 1A). If a decision is made from the received signal that front cover 60 is not open, S1A is repeated.

If from the signal determination portion 167 detects that front cover 60 is opened (YES at S1A), an operation involving indicating a following operation procedure starts. Thus determination portion 167 has a function to detect a timing of starting an operation. CPU 161 initializes a working memory (not shown) of control portion 16 (S2). Subsequently, control portion 166 outputs a signal of instruction through output port 81 and by the signal causes power supply switch LED 51 to flash on and off (S3). Thus the subject is informed (or guided to) that the switch that should first be operated is power supply switch 41. Thus when front cover 60 is opened, an operation guide starts.

Before the subject follows the guide to operate power supply switch 41 (YES at S5), CPU 161 waits for an input received from a switch (S4).

When power supply switch 41 is operated, manipulation detection portion 162 determines from a pulse signal received at input port 71 that power supply switch 41 has been operated (YES at S5).

In accordance with this decision, control portion 166 applies a signal through output port 81 to instruct power supply switch LED 51, which is currently flashes on and off, to turn off. Subsequently a signal is output through output port 82A and 82B to instruct user selection switch LED group 52 to flash on and off each LED (S6) to inform (or guide) the subject (to) that the switch that should next be operated is user selection switch group 42.

Before the subject follows the guide to operate any of user selection switch 42A and 42B and power supply switch 41 (S8), i.e., if a switch other than those switches is operated, CPU 161 waits for an input received from a switch (S7).

Manipulation detection portion 162 detects any of the input ports receives a pulse signal, i.e., determines whether there is any switching operation performed (S8). If any of the input ports receives the pulse signal, a decision is made that input port 71 associated with power supply switch 41 has received the pulse signal. In accordance with the decision CPU 161 ends a series of operations. Thus the current process is terminated. If a decision is made that an input port other than input ports 71, and 72A and 72B has received a pulse signal, i.e., that a switch other than power supply switch 41 and user selection switch group 42 is operated, CPU 161 returns to S7 and waits for an input received from a switch.

If manipulation detection portion 162 determines that input port 72A or 72B receives a pulse signal ("user selection switch" at S8), an operation is performed such as follows: more specifically, if a decision is made that input port 72A receives a pulse signal then in accordance with the decision memory control portion 165 designates the user as "A", i.e., designates memory area 13A as an area at which an obtained measurement is written or an area from which an obtained measurement is recalled. If a decision is made that input port 72B receives a pulse signal, then in accordance with the decision memory control portion 165 designates the user as "B", i.e., designates memory area 13B as an area to which an obtained measurement is written or an area from which an obtained measurement is recalled (S8a).

Subsequently, control portion 166 turns off user selection switch LED group 52 by outputting a signal through output port 82A and 82B to instruct user selection switch LED group 52 to stop flashing on and off. Control portion 166 then outputs a signal through output ports 83 and 84 to instruct measuring switch LED 53 and recalling switch LED 54 to alternately flash on and off (S9). The subject can thus confirm that the switch that should next be operated is measuring switch 43 or recalling switch 44.

With measuring switch 43 flashing on and off (or CPU 161 waiting an input received from a switch), manipulation detection portion 162 determines whether any switch is operated (S 10). This decision is made from whether any input port receives a pulse signal. If any input port receives the pulse signal, manipulation detection portion 162 determines to which switch the pulse signal corresponds (S11). This decision is made from which input port receives the pulse signal. If manipulation detection portion 162 determines that the pulse signal is received at input port 71 ("power supply switch" at S11) then in accordance with the decision CPU 161 instructs power supply portion 15 to stop supplying power to each component. Supplying power by power supply portion 15 to each component thus stops. Accordingly, the LED of each switch of console 4 is also turned off, and a series of operations thus ends.

If manipulation detection portion 162 determines that input port 73 receives a pulse signal ("measuring switch" at S 11) then in accordance with the decision CPU 161 starts a process for measuring blood pressure, as indicated in S12 et. seq., as described later.

If an input port other than input ports 71 and 73 receives a pulse signal, then manipulation detection portion 162 determines that a switch other than measuring switch 43 and power supply switch 41 has been operated ("other switch" at S 11) and in accordance with the decision control portion 166 turns off measuring switch LED 53. Furthermore manipulation detection portion 162 determines the type of the switch operated (S17). More specifically, if the input port having received the pulse signal is input port 72A or 72B, a decision is made that the user (or subject) that has been set in S8 is switched ("user selection switch" at S17). In accordance with the decision, memory control portion 165 switches the current user to that corresponding to the input port receiving the pulse signal (S 18). For example if input port 72A receives the pulse signal the user is set as "A", i.e., memory area 13A is set as an area to which an obtained measurement is written and an area from which an obtained measurement is recalled. If input port 72B receives a pulse signal the user is set as "B", i.e., memory area 13B is set as an area to which an obtained measurement is written and an area from which an obtained measurement is recalled. Thereafter, CPU 161 returns to S 10 and transitions to a state in which it waits until there is an input received from a switch.

In contrast, if manipulation detection portion 162 determines that a pulse signal is received at input port 74 ("recalling switch" at S 17), memory control portion 165 recalls an obtained blood pressure measurement from memory 13 (S 19). More specifically, memory control portion 165 reads data DAi of an obtained measurement from one of memory areas 13A and 13B corresponding to users "A" or "B", respectively, set in S8a or S18, and display control portion 164 causes display 2 to display the read data DAi of the obtained measurement. Thereafter, the control returns to step S10.

Note that at S 19 if morning switch 45 is operated, memory control portion 165 and display control portion 164 recall from a set memory area data DAi of an obtained measurement with the corresponding data DBi of a condition for measurement indicating "morning". In contrast, if evening switch 46 is operated, memory control portion 165 and display control portion 164 recall from a set memory area data DAi of an obtained measurement with the corresponding data DBi of a condition for measurement indicating " evening".

Hereinafter a blood pressure measuring operation performed after S12 et seq. will be described.

The subject operates measuring switch 43 ("measuring switch" at S11). Manipulation detection portion 162 determines from a signal received through input port 73 that measuring switch 43 has been operated. In accordance with the decision, control portion 166 turns off measuring switch LED 53. Subsequently CPU 161 initializes electronic blood pressure monitor 1 by controlling each component to exhaust air internal to the air bag to correct pressure sensor 7 to attain 0 mmHg (S 11a).

When electronic blood pressure monitor is initialized, CPU 161 controls each component to increase the pressure internal to the air bag to the subject's systolic blood pressure or approximately + 40 mmHg (S12) and thereafter gradually decreases the pressure internal to the air bag (S13). In this pressure reduction process the pressure internal to the air bag is detected by pressure sensor 7 and amplification circuit 8, and blood pressure calculation portion 163 receives a voltage signal from amplification circuit 8 and from the received voltage signal calculates a blood pressure (systolic blood pressure and diastolic blood pressure) value and a pulse rate and stores them to CPU 161 at a memory internal thereto temporarily (S14). Display control portion 164 controls display 2 to display the calculated blood pressure value and pulse rate (S 15). The process for increasing and decreasing pressure for measuring blood pressure is similar to that performed by a conventional electronic blood pressure monitor. Note that while herein the electronic blood pressure monitor measures blood pressure in the pressure reduction process, it may do so in the pressurization process.

When blood pressure calculation portion 163 and display control portion 164 complete calculating and displaying a blood pressure and a pulse rate, CPU 161 obtains measurement time data based on time count data provided from timer 14. If the obtained time count data corresponds to a predetermined period of time corresponding to "morning", condition data 13C of memory 13 is set as "morning". If the obtained time count data corresponds to a predetermined period of time corresponding to "evening" then condition data 13C of memory 13 is set as "evening". Then the obtained measurement time data, condition data 13C thus set, and data indicating the value in blood pressure (systolic blood pressure and diastolic blood pressure) and pulse rate read from the memory internal to CPU 161 are used to generate data DAi of an obtained measurement and data DBi of a condition for measurement. Memory control portion 165 associates data DAi and data DBi with each other and stores the associated data to memory area 13A or 13B set in S8a or S18 as an area at which the data are written (S 16). Thus a blood pressure measurement ends.

In Fig. 6, as indicated by S4 and S5 performed in a loop and S7 and S8 performed in a loop, until a subject operates a switch that the subject is informed to next operate, the switch is controlled to continue to flash on and off. In other words, if the subject has operated a switch in an order different from an operation order that (or through which) the subject is informed of (or guided), the subject can be informed of a switch to be operated according to the correct operation order.

### Second Embodiment

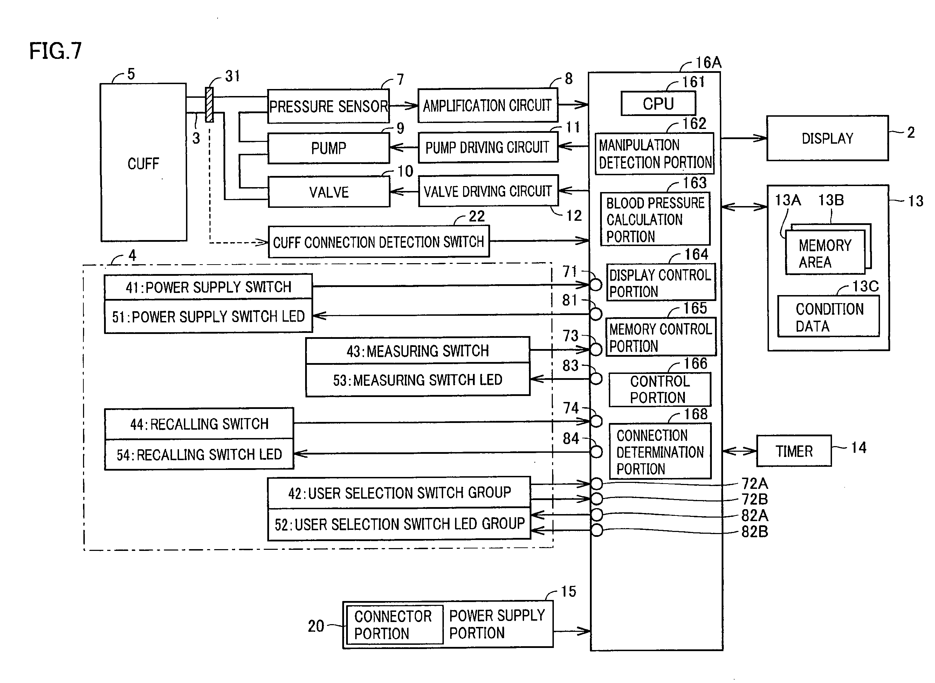

Fig. 7 shows a configuration in function of electronic blood pressure monitor 1 in a second embodiment. It is different from the configuration in function shown in Fig. 4 in that control portion 16 is replaced with a control portion 16A and switch 21 detecting whether front cover 61 is open/closed is replaced with a switch 22 detecting whether cuff 5 is connected. Control portion 16A does not have determination portion 167 determining whether front cover 60 is opened or closed. Instead, control portion 16A has a connection determination portion 168. The remainder of electronic blood pressure monitor 1 is identical to that described in the first embodiment. Connection determination portion 168, as well as determination portion 167 has a function as a detection portion detecting a timing of starting an operation involving guiding a user through an operation procedure.

Cuff 5 is connected to main body 61 via air connector 31 detachably attachably. Switch 22 and connection determination portion 168 configure a cuff detection portion having a function to detect that electronic blood pressure monitor 1 has cuff 5 connected thereto, i.e., attached thereto. Switch 22 is associated with air connector 31 and when switch 22 detects that air connector 31has connected thereto an end of air tube 3 of cuff 51 that is opposite to cuff 5, switch 22 outputs a detection signal to connection determination portion 168. Connection determination portion 168 determines from the detection signal received from cuff connection detection switch 22 whether main body 61 has cuff 5 connected thereto.

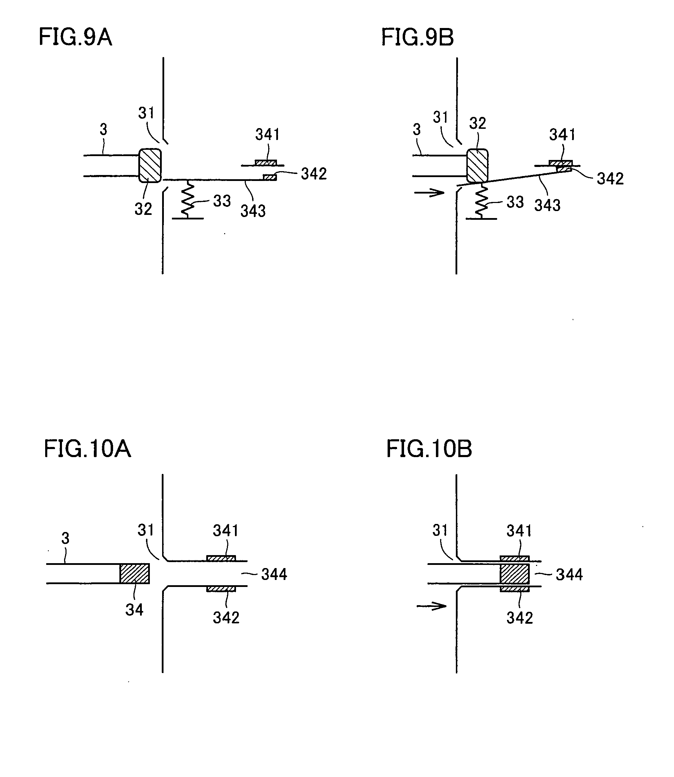

Air connector 31 has electrodes 341 and 342 provided in main body 61 and spaced as predetermined, for example as shown in Figs. 9A and 9B. Electrode 342 is movable upward and downward via a supporting member 343 as a spring 33 is tensioned and compressed. A hole associated with air connector 31 and formed in a side wall of main body 61 externally as an opening receives one end of air tube 3 inserted in a direction indicated by a rightward arrow, as shown in Fig. 9B. Accordingly, spring 33 compressed for example by the weight of a member 32 surrounding one end of air tube 3 and, in reaction thereto, supporting member 343 has that side having electrode 342 mounted thereon moving upward. Thus electrode 342 positionally moves upward and thus contacts electrode 341 and the electrodes establish a short circuit. When switch 22 detects the short circuit, switch 22 outputs to connection determination portion 168 a connection detection signal (transitioning from a level of "0" → a level of "1") indicating that cuff 5 has been connected. While cuff 5 is not connected, the connection detection signal remains at the level of "0". Thus connection determination portion 168 determines that cuff 5 has been connected to main body 61 as the connection detection signal varies in level.

Whether cuff 5 is connected or not may be detected as shown in Figs. 10A and 10B. Internal to main body 61 a path 344 is formed to communicate with an opening provided in a side surface for air connector 31. Path 344 is provided with electrodes 341 and 342 sandwiching path 344. Externally through the opening for air connector 31 path 344 receives an end of air tube 3 inserted thereinto, as indicated in Fig. 10B by an arrow. Air tube 3 is provided at the end with a pipe formed of an electrically conductive member 34. When air tube 3 has the end inserted, electrically conductive member 34 is fit between electrodes 341 and 342 (see Fig. 10B). Accordingly, electrodes 341 and 342 conduct via electrically conductive member 34. When switch 22 detects that the electrodes conduct, switch 22 outputs to connection determination portion 168 the connection detection signal (transitioning from the level of "0" to the level of "1") to indicate that cuff 5 has been connected. While cuff 5 is not connected, the connection detection signal remains at the level of "0". Thus connection determination portion 168 determines that cuff 5 has been connected to main body 61 as the connection detection signal varies in level.

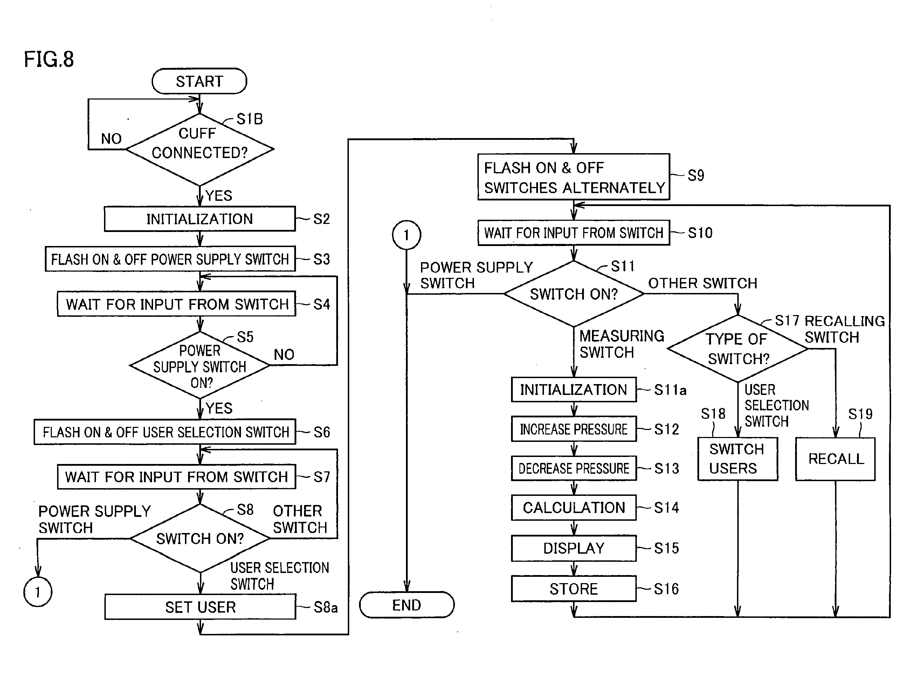

Fig. 8 shows a procedure to inform (guide) a user of (through) an order of operating a manipulation switch to allow electronic blood pressure monitor 1 of the second embodiment to correctly operate (to measure blood pressure and recall measurement data). The Fig. 8 flowchart is stored previously as a program in memory 13 at a predetermined area and read and executed by CPU 161.

The Fig. 8 flowchart differs from the Fig. 6 flowchart in that the Fig. 6 step S1A is replaced with step S1B. The remainder of the process is identical to that shown in Fig. 6.

At step S1B connection determination portion 168 determines from a connection detection signal received from switch 22 whether cuff 5 has been connected to electronic blood pressure monitor 1 at main body 61. More specifically, if the connection detection signal transitions from the level of "0" to the level of "1", connection determination portion 168 responsively determines that cuff 5 has been connected. In response to this decision the process subsequent to S2 et seq. is performed similarly as shown in Fig. 6.

Thus in the second embodiment when cuff 5 is connected to main body 61 an operation guide starts and a switch that should first be operated (i.e., power supply switch 41) flashes on and off.

### Third Embodiment

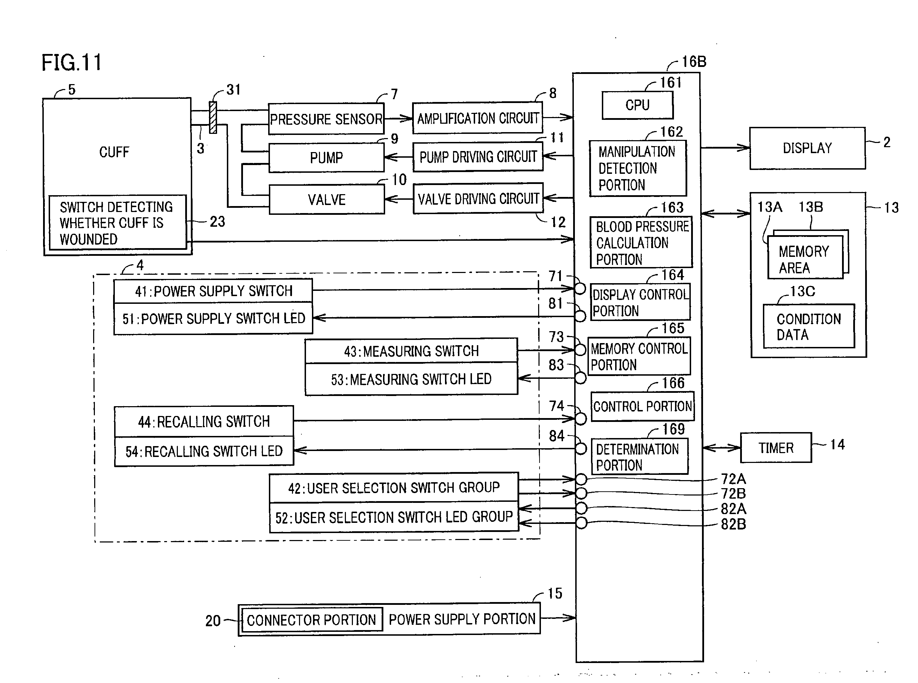

A third embodiment provides electronic blood pressure monitor 1 configured as shown in Fig. 11. The Fig. 11 configuration differs from the Fig. 4 configuration in that control portion 16 is replaced with a control portion 16B and switch 21 detecting whether front cover 60 is opened or closed is replaced with a switch 23 provided internal to cuff 5 to detect whether the cuff is wound around a site for measurement. Control portion 16B does not have determination portion 167 determining whether front cover 60 is opened or closed, as control portion 16 does. Instead, control portion 16B has a determination portion 169 determining whether the cuff has been wound around a site for measurement, and the remainder identical to that of control portion 16. Determination portion 169, as well as determination portion 167, has a function as a detection portion detecting a timing of starting an operation involving guiding a subject through an operation procedure.

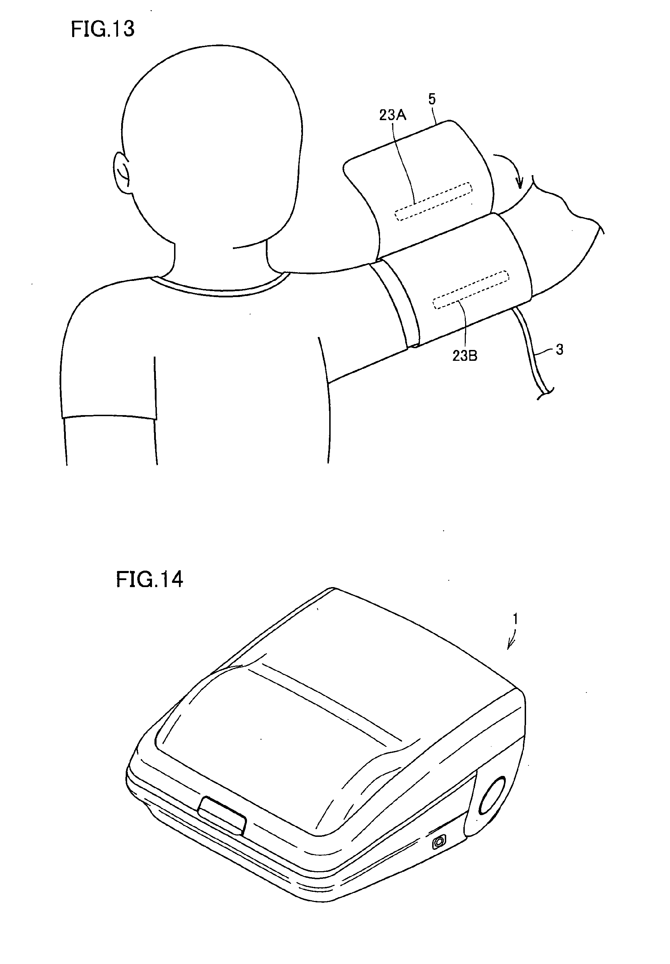

Switch 23 for detecting that cuff 5 has been wound around a site for measurement includes two electrodes 23A and 23B, as shown in Fig. 13. Electrodes 23A and 23B are provided internal to cuff 5. When a subject winds cuff 5 around a site for measurement of blood pressure in a direction indicated by an arrow as shown in Fig. 13 to attach the cuff to the site, electrodes 23A and 23B contact (or establish a short circuit) at a predetermined winding position for measurement. By this short circuit, switch 23 outputs a detection signal transitioning from the level of "0" to the level of "1". The detection signal is received by determination portion 169. Note that it is assumed that when cuff 5 is attached, air tube 3 has been connected to air connector 31.

In response to the detection signal output from switch 23 and transitioning from the level of "0" to the level of "1", determination portion 169 determines that cuff 5 has been attached to the site for measurement of blood pressure. Thus switch 23 and determination portion 169 configure a portion having a function to detect that cuff 5 has been attached to a site for measurement of blood pressure.

Note that while Fig. 11 shows switch 23 having a signal line connected thereto that is connected directly to control portion 16 for the purpose of simplifying the illustration, it is assumed in reality that switch 23 has the signal line connected via tube 3 and air connector 31 to control portion 16.

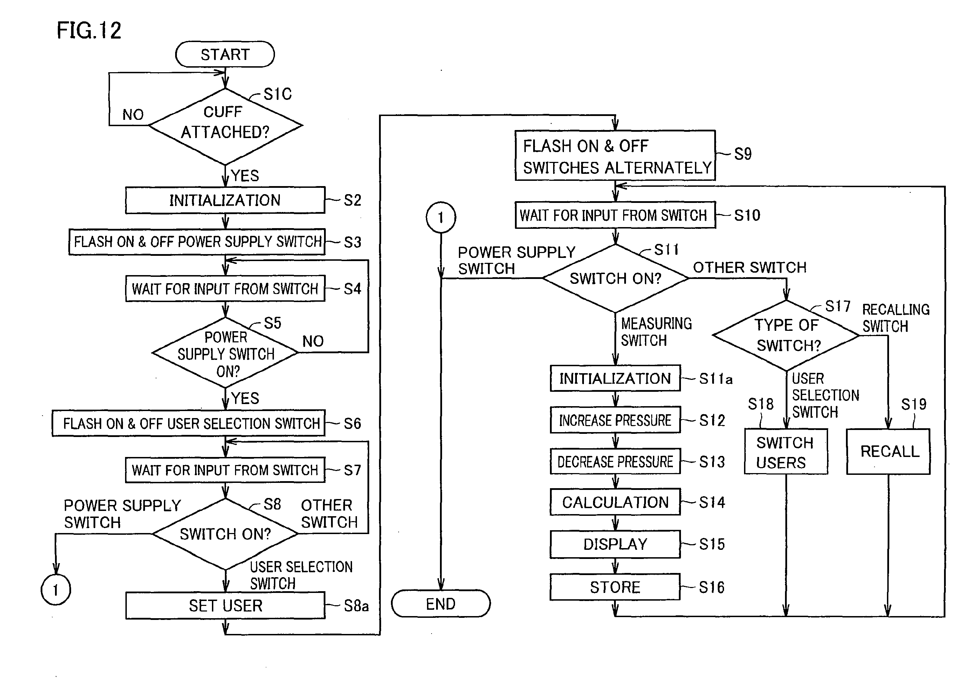

Fig. 12 shows a procedure to inform (guide) a user of (through) an order of operating a manipulation switch to allow electronic blood pressure monitor 1 of the third embodiment to correctly operate (to measure blood pressure and recall measurement data). The Fig. 12 flowchart is stored previously as a program in memory 13 at a predetermined area and read and executed by CPU 161.

The Fig. 12 flowchart differs from the Fig. 6 flowchart in that the Fig. 6 step S1A is replaced with step S1C. The remainder of the process is identical to that shown in Fig. 6.

At step S1C determination portion 169 determines from a detection signal received from switch 23 whether cuff 5 has been wound around (or attached to) a site for measurement of blood pressure. More specifically, if the detection signal transitions from a level of "0" to a level of "1", determination portion 169 responsively determines that cuff 5 has been wound around the site. In response to this decision the process subsequent to S2 et seq. is performed similarly as shown in Fig. 6.

Thus in the third embodiment when cuff 5 is wound around (or attached to) a site for measurement of blood pressure an operation guide starts and a switch that should first be operated (i.e., power supply switch 41) flashes on and off.

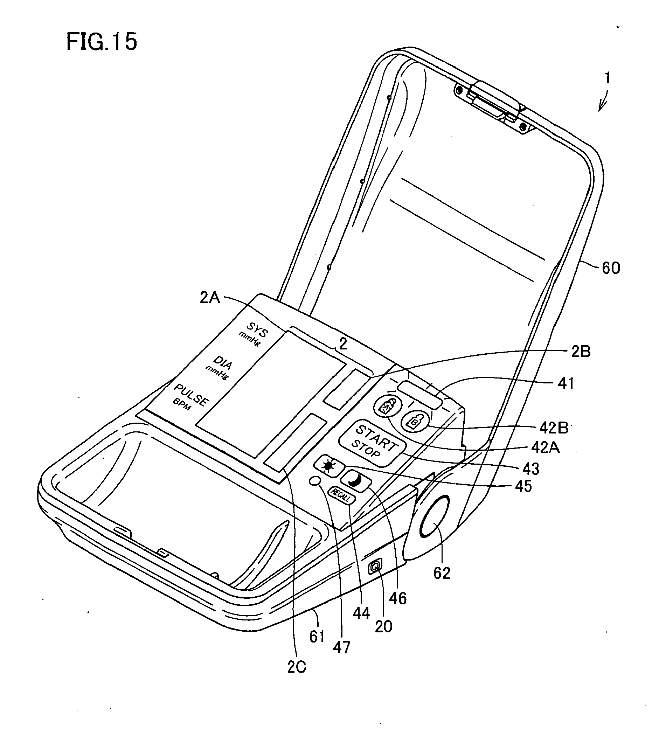

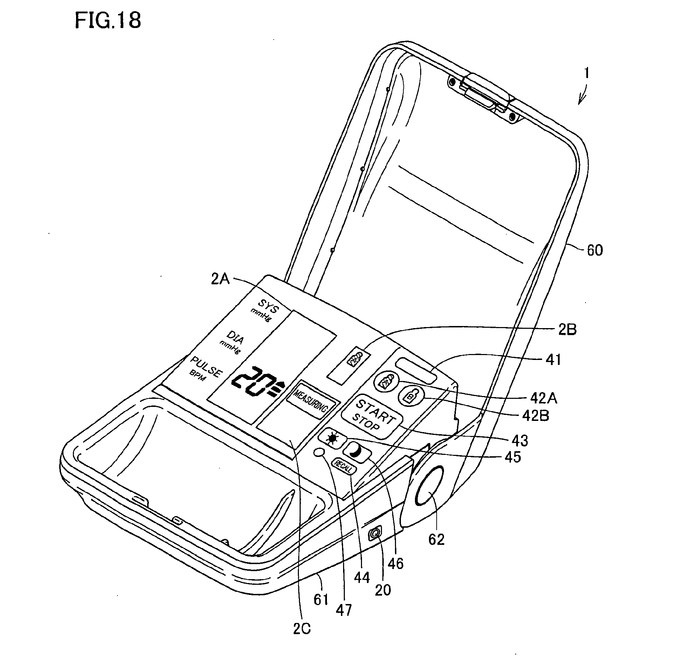

A specific example of informing (guiding) a subject of (through) an operation procedure when an operation guide starts in response to front cover 60 being opened, as described in the first embodiment, will now be described with reference to Figs. 14-18. Note that for the purpose of simplifying illustration Figs. 15-18 do not show main body 61 having cuff 5 connected thereto and power supply cord 18 connected thereto as shown in Fig. 3, although in reality it is assumed that main body 61 has cuff 5 connected thereto and power supply cord 18 connected thereto, as shown in Fig. 3.

Furthermore in Figs. 15-18 display 2 includes an area 2A mainly for displaying data of an obtained measurement, an area 2B for displaying who a subject is, and an area 2C for displaying a type of mode of operation (a blood pressure measuring mode or a measurement data recalling mode). It is assumed that data for displaying in areas 2B and 2C are previously stored for example in memory 13 and read by display control portion 164 and displayed.

Initially a subject prepares electronic blood pressure monitor 1 with front cover 60 closed (i.e., such that cuff 5 and cord reel 19 having power supply cord 18 wound therearound are accommodated), as shown in Fig. 14.

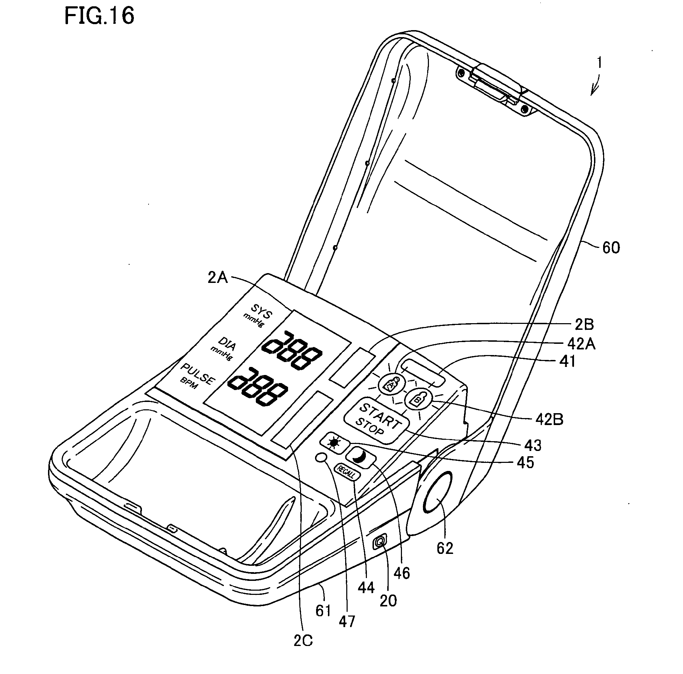

Then to start blood pressure measurement the user opens front cover 60 (see Fig. 15). This causes power supply switch 41 to flash on and off, as has been described in the first embodiment. In this condition, display 2 does not display any indication. Power supply switch 41 flashing on and off guides (or urges) the subject to operate power supply switch 41. Accordingly, display 2 in area 2A displays data, as shown in Fig. 16, to inform the subject that power supply is turned on and each component is supplied with power and ready to operate (to measure blood pressure or recall measurement data). Thus the subject can confirm that in response to power supply switch 41 being operated, each component has been supplied with power.

When power supply switch 41 is operated, CPU 161 stops power supply switch 41 from flashing on and off and causes user selection switch group 42 to flash on and off, as shown in Fig. 16. This informs the subject that the switch to be next operated is either user selection switch 42A or 42B.

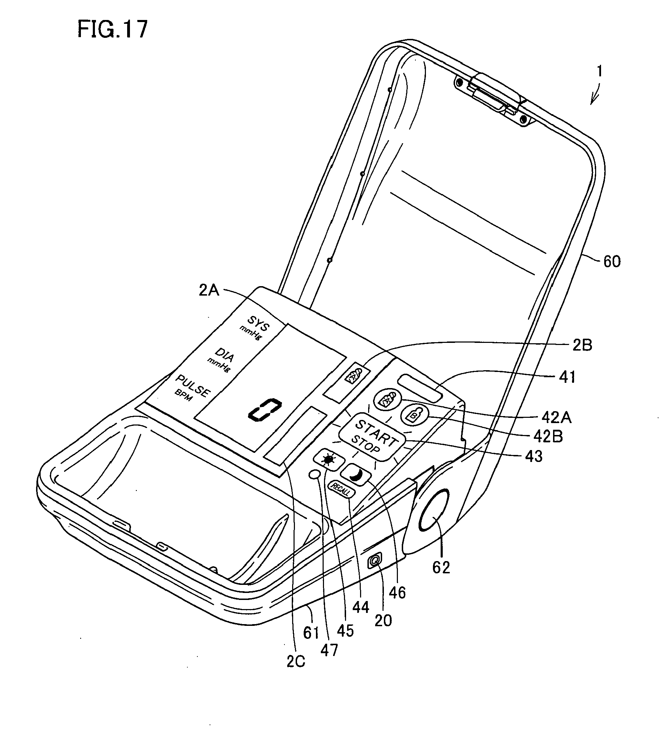

For example if a subject "A" operates user selection switch 42A, control portion 166 stops user selection switch group 42 from flashing on and off and thus turns off user selection switch group 42 and then causes measuring switch 43 to flash on and off. This informs the subject that the switch to be next operated is measuring switch 43. Furthermore display control portion 164 causes display 2 to display "0" in displaying area 2A to indicate that the electronic blood pressure monitor is now ready to operate to measure blood pressure or recall data of an obtained measurement, and display control portion 164 causes display 2 to explicitly indicate in displaying area 2B that "A" has been selected as the user (see Fig. 17). This can inform the subject that a correct user has been selected in electronic blood pressure monitor 1 and that electronic blood pressure monitor 1 is ready to operate.

When the subject operates measuring switch 43 flashing on and off, measuring switch 43 stops flashing on and off and thus turns off and the electronic blood pressure monitor start to measure blood pressure. (Cuff 5 is previously attached to a site for measurement.) When the blood pressure monitor starts to measure blood pressure, display control portion 164 displays in displaying area 2A the data of the blood pressure being measured (or calculated) by blood pressure calculation portion 163. Furthermore, display control portion 164 displays the characters "measuring" in displaying area 2C to indicate that the electronic blood pressure monitor is currently measuring blood pressure. Furthermore, display control portion 164 indicates in area 2B that the subject that is selected to be measurement is "A".

Thus causing display 2 to display in each displaying area the data provided as a switch is operated, can present to a subject how an operation currently proceeds, and also urge the subject to confirm the operation's content(s).

### Still Another Embodiment

In each embodiment as described above console 4 has each switch illuminated by a backlight (the illuminator) implemented by an LED. This can contribute to reduced power consumption and a reduced amount of heat generated. The LED may be replaced with a lamp, although the latter consumes larger power and generates a larger amount of heat than the former.

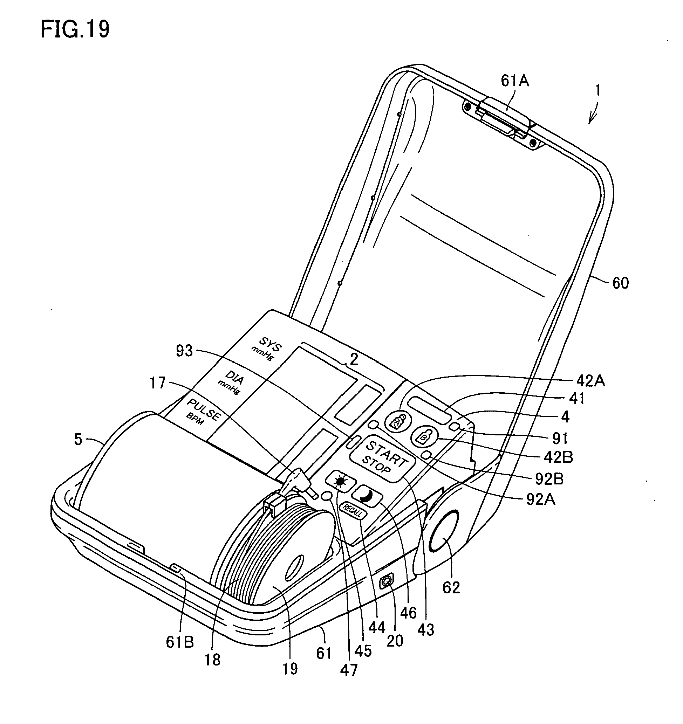

Furthermore, as shown in Fig. 19, the backlight implemented by the LED may be replaced with an illuminator (LEDs 91, 92A, 92B and 93) provided at console 4 in a vicinity of each switch and controlled by control portion 166 via each output port to flash on and off.

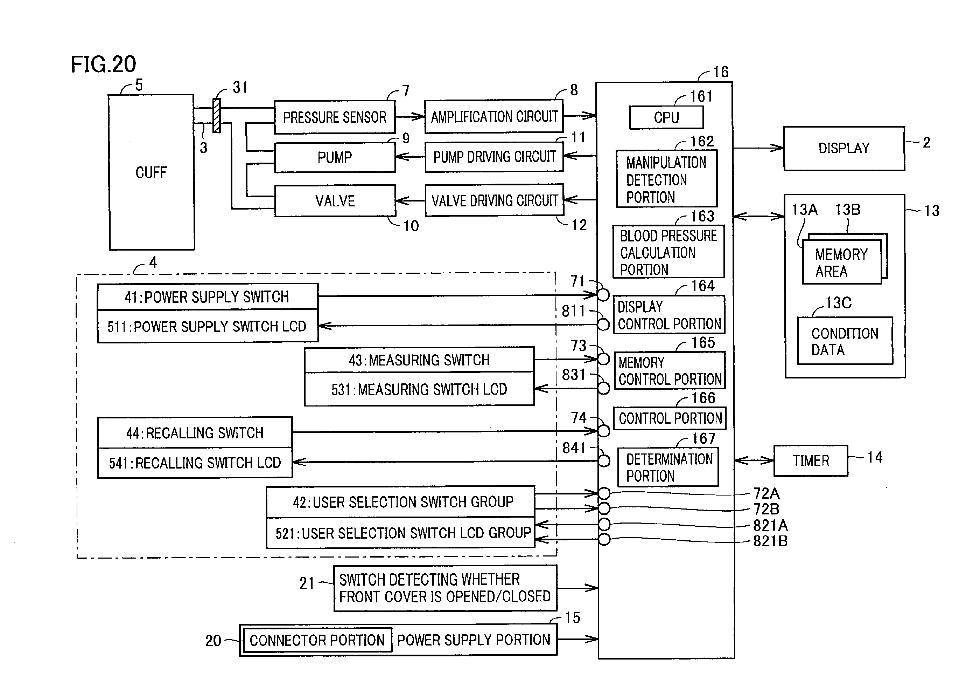

Furthermore, as shown in Fig. 20, the Fig. 4 power supply switch LEDs 51, user selection switch LED group 52, measuring switch LED 93 and recalling switch LED 54 may be replaced with a power supply switch liquid crystal display (LCD) 511, a user selection switch LCD group 521, a measuring switch LCD 531 and a recalling switch LCD 541. These LCDs is controlled to display an indication by a control signal output by control portion 166 through output ports 811, 821A and 821B, 831, and 841. When each LCD displays an indication to indicate that the switch associated with the LCD is a switch to be operated, the LCD may display predetermined information, such as a picture, a character, a symbol or the like, for indicating that the switch is that to be operated. The information displayed by each LCD can be confirmed externally through the light guiding resin material of the switch associated with the LCD and formed to be integrated therewith.

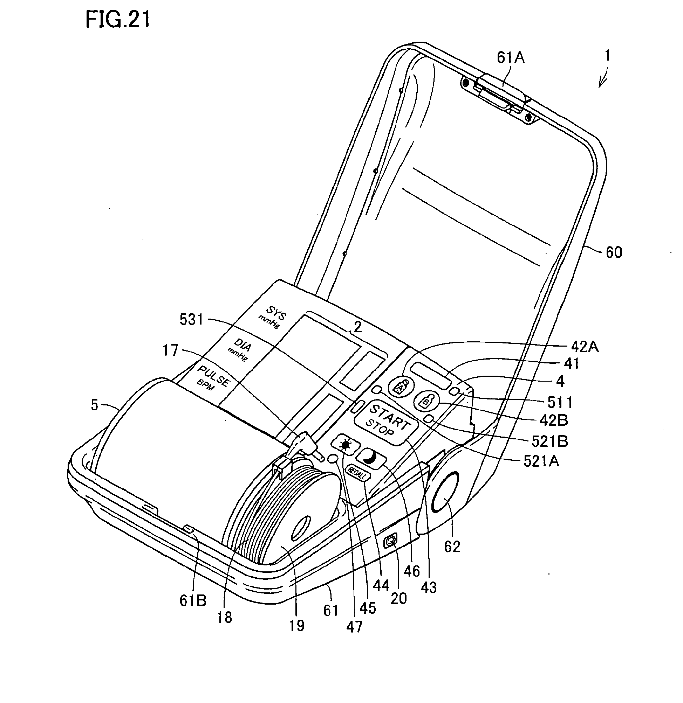

Furthermore, these LCDs may be provided adjacent to their respective manipulation switches, as shown in Fig. 21.

While each embodiment as described above assumes that a plurality of people can share electronic blood pressure monitor 1, it is also applicable if the blood pressure monitor cannot be shared, i.e., if memory 13 does not have a memory area for each individual to store an obtained measurement. In that case, user selection switch group 42 is not provided. Accordingly, measuring switch 43 will be the switch indicated as a switch that should be operated after power supply switch 41 is operated.

Although the present invention has been described and illustrated in detail, it is clearly understood that the same is by way of illustration and example only and is not to be taken by way of limitation, the spirit and scope of the present invention being limited only by the terms of the appended claims.

## Claims

1. An electronic blood pressure monitor (1) comprising:
a cuff (5) attached at a site for measurement of blood pressure;
a pressure adjustment unit (9, 10) adjusting a pressure applied to said cuff;
a pressure detection unit (7) detecting a pressure internal to said cuff;
a blood pressure calculation unit (163) calculating a blood pressure based on a detection signal output from said pressure detection unit;
a storage portion (13) storing to a memory said blood pressure calculated by said blood pressure calculation unit;
a display (2) at least displaying said blood pressure calculated by said blood pressure calculation unit; and
a plurality of manipulation portions (4) each externally operated to indicate an operation of the electronic blood pressure monitor, wherein to indicate said operation an order of operating said manipulation portion to be operated is indicated in a predetermined manner.

2. The electronic blood pressure monitor according to claim 1, further comprising a cover covering at least a portion of the electronic blood pressure monitor and opened and closed as desired, wherein when said cover is opened, one of said plurality of manipulation portions that should first be operated is indicated in said predetermined manner.

3. The electronic blood pressure monitor according to claim 2, further comprising a joint jointing said cover and a main body of the electronic blood pressure monitor together, wherein to allow said cover to be opened and closed as desired, said cover is jointed via said joint to said main body pivotably and whether said cover is opened or closed is detected from whether said cover pivots.

4. The electronic blood pressure monitor according to claim 2, wherein whether the main body of the electronic blood pressure monitor and said cover contact each other is detected to detect whether said cover is open or closed.

5. The electronic blood pressure monitor according to claim 1, said cuffbeing detachable to the electronic blood pressure monitor, further comprising a cuff detection unit detecting whether the electronic blood pressure monitor has said cuff attached thereto, wherein when said cuff detection unit detects that the electronic blood pressure monitor has said cuff attached thereto, one of said plurality of manipulation portions that should first be operated is indicated in said predetermined manner.

6. The electronic blood pressure monitor according to claim 1, further comprising a detection unit detecting that said cuff has been attached to said site for measurement, wherein when said detection unit detecting that said cuff has been attached to said site for measurement detects that said cuff has been attached to said site for measurement, one of said plurality of manipulation portions that should first be operated is indicated in said predetermined manner.

7. The electronic blood pressure monitor according to claim 1, wherein when said manipulation portion is operated in an order different from said order indicated, said manipulation portion that should be operated in accordance with said order indicated is indicated in said predetermined manner.

8. The electronic blood pressure monitor according to claim 1, wherein said predetermined manner is illuminating said manipulation portion.

9. The electronic blood pressure monitor according to claim 8, further comprising an illuminator associated with each of said plurality of manipulation portions and provided to be integrated therewith for illuminating said manipulation portion.

10. The electronic blood pressure monitor according to claim 9, wherein said illuminator associated with said manipulation portion to be operated in said order indicated is turned on or flashed on and off in said order indicated.

11. The electronic blood pressure monitor according to claim 8, further comprising an illuminator associated with each of said plurality of manipulation portions and provided in a vicinity of said manipulation portion for illuminating said manipulation portion.

12. The electronic blood pressure monitor according to claim 11, wherein said illuminator associated with said manipulation portion to be operated in accordance with said order indicated is turned on or flashed on and off in said order indicated.

13. The electronic blood pressure monitor according to claim 1, further comprising an information displaying portion associated with each of said plurality of manipulation portions to display predetermined information, wherein said information displaying portion that is associated with said manipulation portion to be operated in accordance with said order indicated displays said predetermined information in said order indicated.

14. The electronic blood pressure monitor according to claim 13, wherein said manipulation portion and said information displaying portion associated therewith are integrally configured.

15. The electronic blood pressure monitor according to claim 13, wherein said information displaying portion is arranged in a vicinity of said manipulation portion associated therewith.

16. A method of indicating an order of operating an electronic blood pressure monitor (1) including
a cuff (5) attached at a site for measurement of blood pressure,
a pressure adjustment unit (9, 10) adjusting a pressure applied to said cuff,
a pressure detection unit (7) detecting a pressure internal to said cuff,
a blood pressure calculation unit (163) calculating a blood pressure based on a detection signal output from said pressure detection unit,
a storage portion (13) storing to a memory said blood pressure calculated by said blood pressure calculation unit;
a display (2) at least displaying said blood pressure calculated by said blood pressure calculation unit, and
a plurality of manipulation portions (4) each externally operated to indicate an operation of the electronic blood pressure monitor, the method comprising the steps of:
detecting a timing of starting to indicate said order of operating the electronic blood pressure monitor; and
in response to said timing being detected in the step of detecting, to indicate said operation, indicating in a predetermined manner an order of operating said manipulation portion to be operated.
